Europäisches Patentamt
European Patent Office
Office européen des brevets

(11) Veröffentlichungsnummer: **0 362 606 A1**

# EUROPÄISCHE PATENTANMELDUNG

(21) Anmeldenummer: 89117180.3

(22) Anmeldetag: 16.09.89

(51) Int. Cl.5: C07D 233/56 , A01N 43/56 , C07D 231/16 , A01N 43/50 , A01N 43/653 , C07D 249/08

(30) Priorität: 01.10.88 DE 3833549

(43) Veröffentlichungstag der Anmeldung: 11.04.90 Patentblatt 90/15

(84) Benannte Vertragsstaaten: BE CH DE FR GB IT LI NL

(71) Anmelder: BAYER AG

D-5090 Leverkusen 1 Bayerwerk(DE)

(72) Erfinder: Haug, Michael, Dr.
Fahner Weg 5
D-5060 Bergisch Gladbach 2(DE)
Erfinder: Pfister, Theodor, Dr.
Lichtenberger Strasse 30
D-4019 Monheim(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Lürssen, Klaus, Dr.
August-Kierspel-Strasse 145
D-5060 Bergisch Gladbach 2(DE)
Erfinder: Strang, Harry, Dr.
Heiderweg 53
D-4000 Düsseldorf 31(DE)

## (54) Substituierte Phenoxyphenylsulfonylazole, Verfahren zu ihrer Herstellung sowie ihre Verwendung als Herbizide und Pflanzenwuchsregulatoren.

(57) Die Erfindung betrifft neue substituierte Phenoxyphenylsulfonylazole der Formel (I)

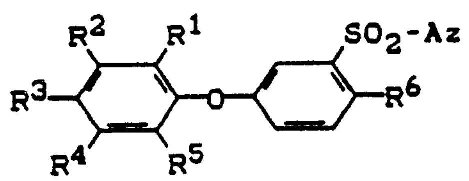

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls substituiertes Azolyl steht,
Verfahren zur ihrer Herstellung sowie ihre Verwendung als Herbizide und Pflanzenwuchsregulatoren.

EP 0 362 606 A1

## Substituierte Phenoxyphenylsulfonylazole, Verfahren zu ihrer Herstellung sowie ihre Verwendung als Herbizide und Pflanzenwuchsregulatoren

Die Erfindung betrifft neue substituierte Phenoxyphenylsulfonylazole, Verfahren zu ihrer Herstellung sowie ihre Verwendung als Herbizide und Pflanzenwuchsregulatoren.

Es ist bekannt, daß bestimmte Phenoxyphenylverbindungen, wie z. B. 3-(2,4-Dichlor-phenoxy)-6-nitrobenzoesäuremethylester (Bifenox) herbizid wirksam sind (vgl. US-P 3 652 645 und US-P 3 776 715). Die Wirkung dieser bekannten Verbindung ist jedoch nicht in allen Belangen zufriedenstellend.

Es wurden nun neue substituierte Phenoxyphenylsulfonylazole der allgemeinen Formel (I)

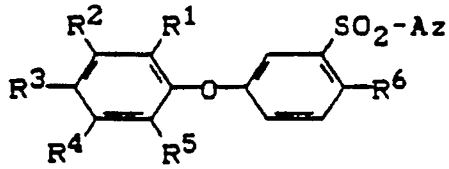

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls substituiertes Azolyl steht,
gefunden.

Weiter wurde gefunden, daß man die neuen substituierten Phenoxyphenylsulfonylazole der allgemeinen Formel (I) erhält, wenn man

(a) für den Fall, daß in Formel (I) $R^6$ für Wasserstoff oder Nitro steht und $R^1$, $R^2$, $R^3$, $R^4$, $R^5$ und Az die oben angegebenen Bedeutungen haben,
substituierte Phenoxybenzolsulfonsäurechloride der allgemeinen Formel (II)

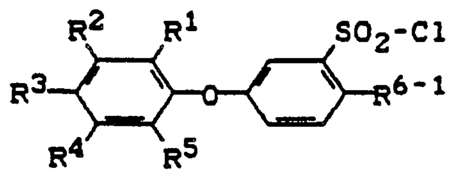

(II)

in welcher
$R^{6-1}$ für Wasserstoff oder Nitro steht und
$R^1$, $R^2$, $R^3$, $R^4$ und $R^5$ die oben angegebenen Bedeutungen haben,
mit Azolen der allgemeinen Formel (III)
H - Az (III)
in welcher
Az die oben angegebene Bedeutung hat,
oder mit Alkalimetallsalzen von Azolen der Formel (III)
gegebenenfalls in Gegenwart eines Säureakzeptors und gegebenenfalls in Gegenwart eines Verdünnungsmittels umsetzt, oder wenn man

(b) für den Fall, daß in Formel (I) $R^6$ für Amino steht und $R^1$, $R^2$, $R^3$, $R^4$, $R^5$ und Az die oben angegebenen Bedeutungen haben,
Verbindungen der allgemeinen Formel (I), in welcher $R^6$ für Nitro steht und $R^1$, $R^2$, $R^3$, $R^4$, $R^5$ und Az die oben angegebenen Bedeutungen haben,
mit Wasserstoff in Gegenwart eines Hydrierungskatalysators und in Gegenwart eines Verdünnungsmittels umsetzt, oder wenn man

(c) für den Fall, daß in Formel (I) $R^6$ für Halogen oder Cyano steht und $R^1$, $R^2$, $R^3$, $R^4$, $R^5$ und Az die oben angegebenen Bedeutungen haben,

Verbindungen der allgemeinen Formel (I), in welcher $R^6$ für Amino steht und $R^1$, $R^2$, $R^3$, $R^4$, $R^5$ und Az die oben angegebenen Bedeutungen haben,
mit Natriumnitrit oder Kaliumnitrit und mit einem Hydrogenhalogenid (für $R^6$: Halogen) bzw. mit Schwefelsäure (für $R^6$: Cyano) in Gegenwart von Wasser und gegebenenfalls in Gegenwart eines organischen Lösungsmittels umsetzt und die hierbei erhaltenen Diazoniumsalzlösungen mit in Wasser gelösten Kupfer-(I)-halogeniden bzw. mit Kupfer(I)-cyanid umsetzt.

Schließlich wurde gefunden, daß die neuen substituierten Phenoxyphenylsulfonylazole der Formel (I) starke herbizide and pflanzenwuchsregulierende Wirkung zeigen.

Überraschenderweise sind die erfindungsgemäßen substituierten Phenoxyphenylsulfonylazole der Formel (I) gegen Unkräuter wesentlich stärker wirksam als 3-(2,4-Dichlorphenoxy)-6-nitro-benzoesäure-methylester, welches ein strukturell ähnlicher vorbekannter Wirkstoff gleicher Wirkungsrichtung ist.

Die Erfindung betrifft vorzugsweise Verbindungen der Formel (I), in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und Halogen-$C_1$-$C_4$-alkylthio substituiertes Pyrazolyl, Imidazolyl oder Triazolyl steht.

Die Erfindung betrifft besonders bevorzugt Verbindungen der Formel (I), in welcher
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$ für Wasserstoff, Fluor, Chlor oder Brom steht,
$R^5$ für Wasserstoff, Fluor, Chlor oder Brom steht,
$R^6$ für Wasserstoff, Fluor, Chlor, Brom, Iod, Cyano, Nitro oder Amino steht und
Az gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und Halogen-$C_1$-$C_4$-alkylthio substituiertes Pyrazolyl, Imidazolyl oder Triazolyl steht.

Die Erfindung betrifft insbesondere Verbindungen der Formel (I), in welcher
$R^1$ für Wasserstoff, Fluor oder Chlor steht,
$R^2$ für Wasserstoff, Fluor oder Chlor steht,
$R^3$ für Trifluormethyl steht,
$R^4$ für Wasserstoff, Fluor oder Chlor steht,
$R^5$ für Wasserstoff, Fluor oder Chlor steht,
$R^6$ für Wasserstoff, Chlor, Brom, Cyano, Nitro oder Amino steht und
Az für gegebenenfalls einfach bis dreifach, gleich oder verschieden durch Chlor, Brom, Methyl, Ethyl und Trifluormethyl substituiertes Pyrazolyl oder Imidazolyl steht.

Beispiele für die substituierten Phenoxyphenylsulfonylazole sind in der nachstehenden Tabelle 1 aufgeführt.

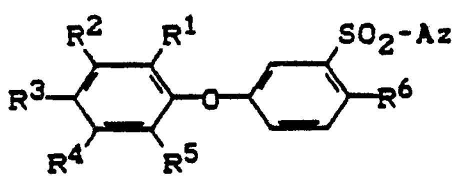

(I)

Tabelle 1

| $R^1$ | $R^2$ | $R^3$ | $R^4$ | $R^5$ | $R^6$ | Az |
|---|---|---|---|---|---|---|
| Cl | H | $CF_3$ | H | H | $NO_2$ | 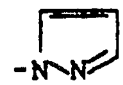 |
| Cl | H | $CF_3$ | H | Cl | $NO_2$ | 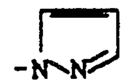 |
| Cl | H | $CF_3$ | H | F | $NO_2$ | 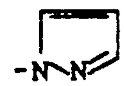 |
| Cl | H | $CF_3$ | Cl | Cl | $NO_2$ | 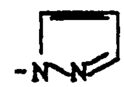 |
| Cl | H | $CF_3$ | F | Cl | $NO_2$ | 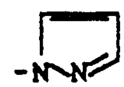 |
| Cl | H | $CF_3$ | H | Cl | $NO_2$ | 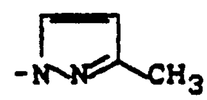 |
| Cl | H | $CF_3$ | H | F | $NO_2$ | 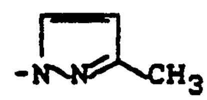 |

Tabelle 1 - Fortsetzung

| $R^1$ | $R^2$ | $R^3$ | $R^4$ | $R^5$ | $R^6$ | Az |
|---|---|---|---|---|---|---|
| Cl | H | $CF_3$ | H | Cl | $NO_2$ | 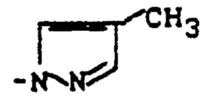 |
| Cl | H | $CF_3$ | H | F | $NO_2$ | 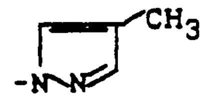 |
| Cl | H | $CF_3$ | H | Cl | $NO_2$ | 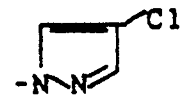 |
| Cl | H | $CF_3$ | H | Cl | $NO_2$ | 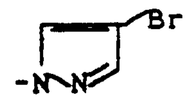 |
| Cl | H | $CF_3$ | H | H | $NO_2$ | 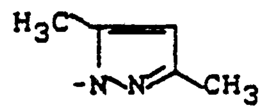 |
| Cl | H | $CF_3$ | H | Cl | $NO_2$ | 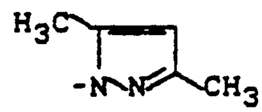 |
| Cl | H | $CF_3$ | H | F | $NO_2$ | 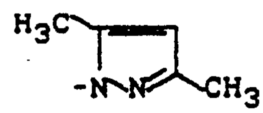 |
| Cl | H | $CF_3$ | H | Cl | $NO_2$ | 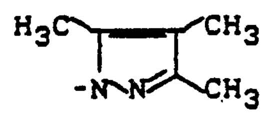 |
| Cl | H | $CF_3$ | H | F | $NO_2$ | 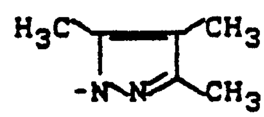 |
| Cl | H | $CF_3$ | H | H | $NO_2$ | 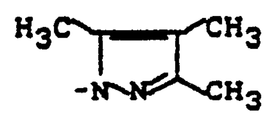 |

Tabelle 1 - Fortsetzung

| $R^1$ | $R^2$ | $R^3$ | $R^4$ | $R^5$ | $R^6$ | Az |
|---|---|---|---|---|---|---|
| Cl | H | $CF_3$ | H | H | $NO_2$ | 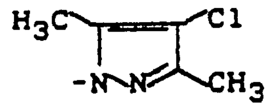 |
| Cl | H | $CF_3$ | H | Cl | $NO_2$ | 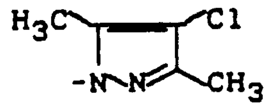 |
| Cl | H | $CF_3$ | H | F | $NO_2$ | 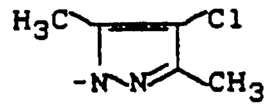 |
| Cl | H | $CF_3$ | H | H | $NO_2$ | 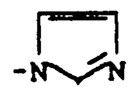 |
| Cl | H | $CF_3$ | H | Cl | $NO_2$ | 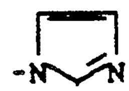 |
| Cl | H | $CF_3$ | H | F | $NO_2$ | 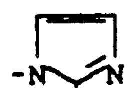 |
| Cl | H | $CF_3$ | H | F | $NO_2$ | 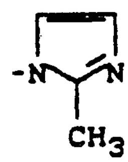 |
| Cl | H | $CF_3$ | H | H | $NO_2$ | 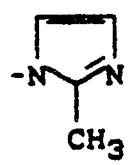 |
| Cl | H | $CF_3$ | H | Cl | $NO_2$ | 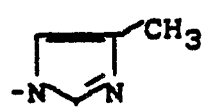 |

Tabelle 1 - Fortsetzung

| $R^1$ | $R^2$ | $R^3$ | $R^4$ | $R^5$ | $R^6$ | Az |
|---|---|---|---|---|---|---|
| Cl | H | $CF_3$ | H | H | $NH_2$ | 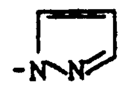 |
| Cl | H | $CF_3$ | H | Cl | $NH_2$ | 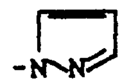 |
| Cl | H | $CF_3$ | H | F | $NH_2$ | 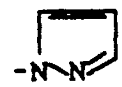 |
| Cl | H | $CF_3$ | Cl | Cl | $NH_2$ | 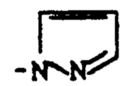 |
| Cl | H | $CF_3$ | F | Cl | $NH_2$ | 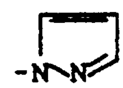 |
| Cl | H | $CF_3$ | H | Cl | $NH_2$ | 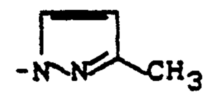 |
| Cl | H | $CF_3$ | H | F | $NH_2$ | 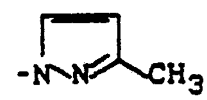 |
| Cl | H | $CF_3$ | H | Cl | $NH_2$ | 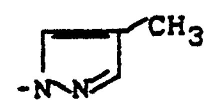 |
| Cl | H | $CF_3$ | H | F | $NH_2$ | 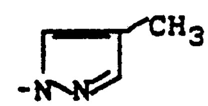 |
| Cl | H | $CF_3$ | H | Cl | $NH_2$ | 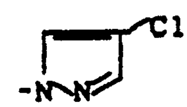 |

Tabelle 1 - Fortsetzung

| $R^1$ | $R^2$ | $R^3$ | $R^4$ | $R^5$ | $R^6$ | Az |
|---|---|---|---|---|---|---|
| Cl | H | $CF_3$ | H | Cl | $NH_2$ | 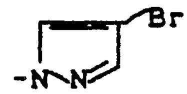 |
| Cl | H | $CF_3$ | H | H | $NH_2$ | 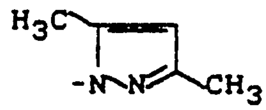 |
| Cl | H | $CF_3$ | H | Cl | $NH_2$ | 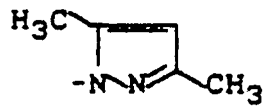 |
| Cl | H | $CF_3$ | H | F | $NH_2$ | 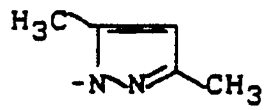 |
| Cl | H | $CF_3$ | H | Cl | $NH_2$ | 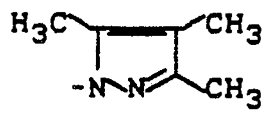 |
| Cl | H | $CF_3$ | H | F | $NH_2$ | 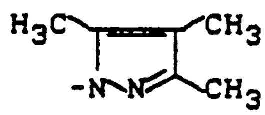 |
| Cl | H | $CF_3$ | H | H | $NH_2$ | 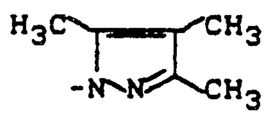 |
| Cl | H | $CF_3$ | H | H | $NH_2$ | 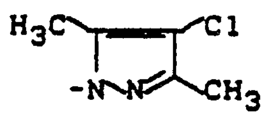 |
| Cl | H | $CF_3$ | H | Cl | $NH_2$ | 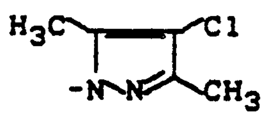 |
| Cl | H | $CF_3$ | H | F | $NH_2$ | 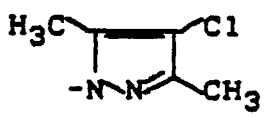 |

Tabelle 1 - Fortsetzung

| $R^1$ | $R^2$ | $R^3$ | $R^4$ | $R^5$ | $R^6$ | Az |
|---|---|---|---|---|---|---|
| Cl | H | $CF_3$ | H | H | $NH_2$ | 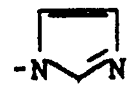 |
| Cl | H | $CF_3$ | H | Cl | $NH_2$ | 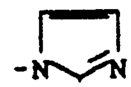 |
| Cl | H | $CF_3$ | H | F | $NH_2$ | 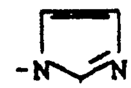 |
| Cl | H | $CF_3$ | H | F | $NH_2$ | 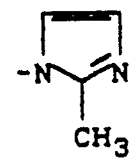 |
| Cl | H | $CF_3$ | H | H | $NH_2$ | 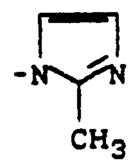 |
| Cl | H | $CF_3$ | H | Cl | $NH_2$ | 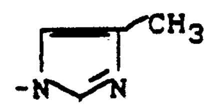 |

Verwendet man für das erfindungsgemäße Verfahren (a) beispielsweise 2-Nitro-5-(2,6-difluor-4-trifluormethylphenoxy)-benzolsulfonsäurechlorid und 2-Methyl-imidazol als Ausgangsstoffe, so kann der Reaktionsablauf durch das folgende Formelschema wiedergegeben werden:

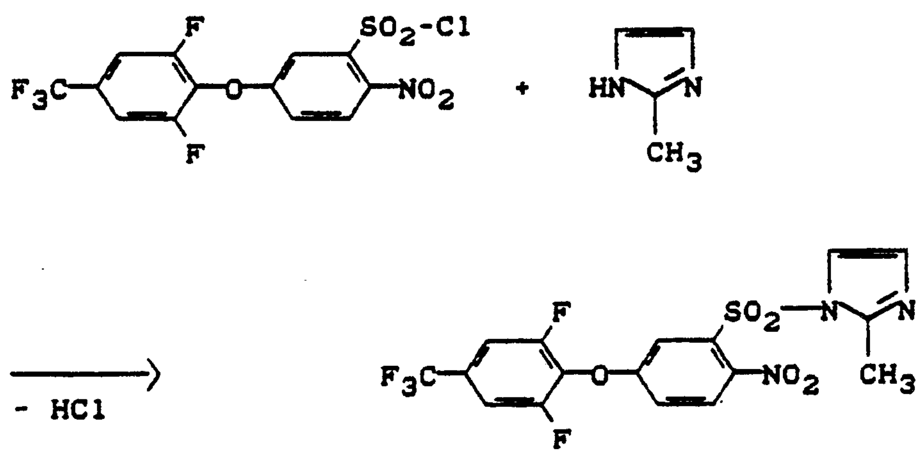


Verwendet man für das erfindungsgemäße Verfahren (b) beispielsweise (2-Nitro-5-(2-chlor-4-trifluormethylphenoxy)-phenyl-sulfonylpyrazol und Wasserstoff in Gegenwart eines Platin-Katalysators als Ausgangsstoffe, so kann der Reaktionsablauf durch des folgende Formelschema wiedergegeben werden:

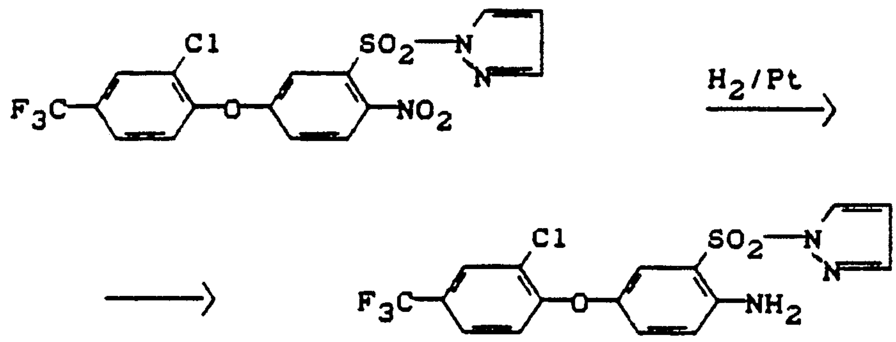


Verwendet man für das erfindungsgemäße Verfahren (c) beispieleweise (2-Amino-5-(2-chlor-6-fluor-4-trifluormethyl-phenoxy)-phenyl-sulfonylpyrazol und Natriumnitrit /Hydrogenbromid sowie anschließend Kupfer(I)-bromid als Ausgangsstoffe, so kann der Reaktionsablauf durch das folgende Formelschema wiedergegeben werden:

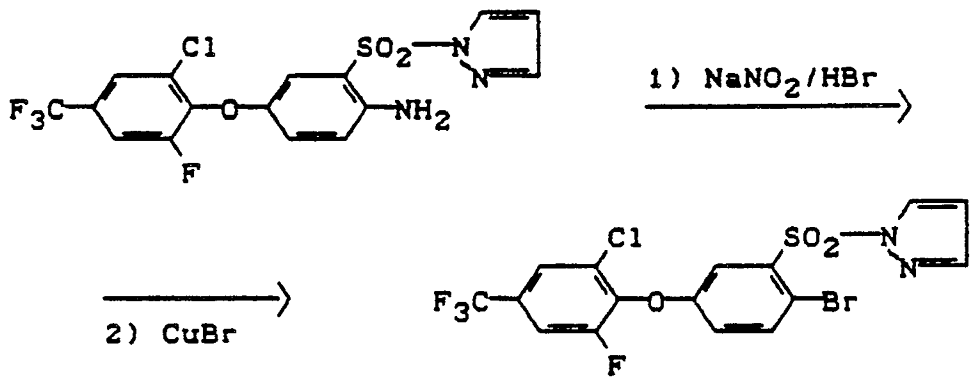


Die beim erfindungsgemäßen Verfahren (a) zur Herstellung von Verbindungen der Formel (I) als Ausgangsstoffe zu verwendenden substituierten Phenoxybenzolsulfonsäurechloride sind durch die Formel (II) allgemein definiert.

In Formel (II) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden und $R^{6-1}$ steht für Wasserstoff oder Nitro.

Als Beispiele für die Ausgangsstoffe der Formel (II) seien genannt:
3-(2-Chlor-4-trifluormethyl-phenoxy)-, 3-(2,6-Dichlor-4-trifluormethyl-phenoxy)-, 3-(2-Chlor-6-fluor-4-trifluormethyl-phenoxy)-,3-(2,3,6-Trichlor-4-trifluormethyl)-phenoxy)- und 3-(2,6-Dichlor-3-fluor-4-trifluormethyl-phenoxy)-benzolsulfonsäurechlorid; 2-Nitro-5-(2-chlor-4-trifluormethyl-phenoxy)-, 2-Nitro-5-(2,6-dichlor-4-trifluormethyl-phenoxy-, 2-Nitro-5-(2-chlor-6-fluor-4-trifluormethyl-phenoxy)-, 2-Nitro-5-(2,3,6-trichlor-4-trifluormethyl-phenoxy)- und 2-Nitro-5-(2,6-dichlor-3-fluor-4-trifluormethyl-phenoxy)-benzolsulfonsäurechlorid.

Die Ausgangsstoffe der Formel (II) sind bekannt und/oder können nach an sich bekannten Verfahren hergestellt werden (vgl. US-P 4 213 775).

Man erhält die Verbindungen der Formel (II) beispielsweise, wenn man

(α) für den Fall, daß $R^{6-1}$ für Wasserstoff steht,

substituierte Aminodiphenylether der allgemeinen Formel (IV)

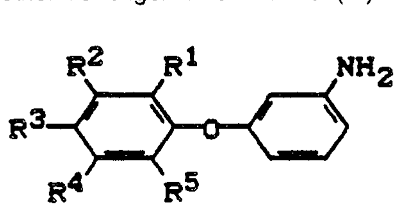

(IV)

in welcher
R',$R^2$,$R^3$,$R^4$ und $R^5$ die oben angegebenen Bedeutungen haben,
mit Natriumnitrit oder Kaliumnitrit in Gegenwart wäßriger Salzsäure und gegebenenfalls in Gegenwart eines organischen Lösungsmittels, wie z. B. Essigsäure, bei Temperaturen zwischen -10 °C und +20 °C umsetzt und die hierbei erhaltenen Diazoniumsalzlösungen mit Schwefeldioxid in Gegenwart eines Katalysators, wie z. B. Kupfer(I)- und/oder Kupfer(II)-chlorid, bei Temperaturen zwischen -10 °C und +40 °C umsetzt, oder wenn man

($\beta$) für den Fall, daß $R^{6-1}$ für Nitro steht,
substituierte Dinitrodiphenylether der allgemeinen Formel (V)

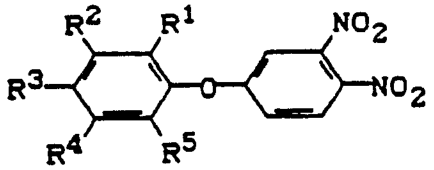
(V)

in welcher
$R^1$, $R^2$, $R^3$, $R^4$ und $R^5$ die oben angegebenen Bedeutungen haben,
mit Benzylmercaptan, gegebenenfalls in Gegenwart eines Säureakzeptors, wie z. B. Kaliumhydroxid, und gegebenenfalls in Gegenwart von Verdünnungsmitteln, wie z. B. Wasser, Ethanol und Isopropanol, bei Temperaturen zwischen 0 °C und 100 °C umsetzt, die hierbei erhaltenen substituierten Benzylthionitrodiphenylether der allgemeinen Formel (VI)

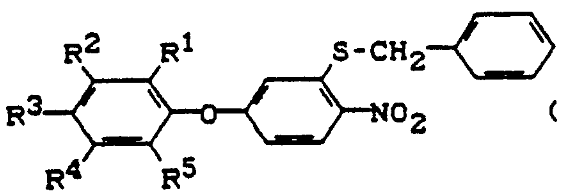
(VI)

in welcher
$R^1$, $R^2$, $R^3$, $R^4$ und $R^5$ die oben angegebenen Bedeutungen haben,
durch Absaugen isoliert und mit Chlor in Gegenwart von Verdünnungsmitteln, wie z. B. Wasser und Essigsäure, bei Temperaturen zwischen 0 °C und 50 °C umsetzt.

Die Zwischenprodukte der Formeln (IV) und (V) sind bekannt und/oder können nach an sich bekannten Verfahren hergestellt werden (vgl. EP-A 29 123; EP-A 7 471).

Die beim erfindungsgemäßen Verfahren (a) weiter als Ausgangsstoffe zu verwendenen Azole sind durch die Formel (III) allgemein definiert. In Formel (III) hat Az vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Az angegeben wurde. Bevorzugte Alkalimetallsalze der Azole der Formel (III) sind deren Lithium-, Natrium- oder Kaliumsalze.

Als Beispiele für die Ausgangsstoffe der Formel (III) seien genannt:
Pyrazol, 4-Chlor-pyrazol, 4-Brom-pyrazol, 3-Methyl-, 4-Methyl-, 3,5-Dimethyl- und 3,4,5-Trimethyl-pyrazol, 4-Chlor-3,5-dimethyl-pyrazol, Imidazol, 2-Methyl- und 4-Methyl-imidazol.

Die Verbindungen der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Hep tan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (a) alle üblicherweise für derartige

Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und - alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicycylo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerüht. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden Verbindungen sind - mit der Maßgabe, daß jeweils $R^6$ für Nitro steht - durch die Formel (I) allgemein definiert.

In Formel (I) haben dann $R^1$, $R^2$, $R^3$, $R^4$, $R^5$ und Az vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und Az angegeben wurden.

Die Ausgangsstoffe der Formel (I) für Verfahren (b) sind erfindungsgemäße, neue Verbindungen; sie können nach den erfindungsgemäßen Verfahren (a) hergestellt werden.

Verfahren (b) wird unter Verwendung eines Hydrierungskatalysators durchgeführt. Als Beispiele hierfür seien Raney-Nickel, Platin und Palladium genannt. Vorzugsweise wird Raney-Nickel für Verfahren (b) eingesetzt.

Verfahren (b) wird in Gegenwart eines Verdünnungsmittels durchgeführt. Vorzugsweise werden Alkohole, wie Methanol, Ethanol, Propanol oder Isopropanol, Ether, wie Glycoldimethylether, Diglycoldimethylether, Tetrahydrofuran oder Dioxan, oder Ester, wie Essigsäuremethylester oder Essigsäureethylester, als Lösungsmittel für Verfahren (b) eingesetzt.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (b) wird im allgemeinen bei Normaldruck oder erhöhtem Druck bis etwa 200 bar, vorzugsweise bis etwa 100 bar, durchgeführt.

Verfahren (b) kann unter den bei katalytischen Hydrierungen üblichen Bedingungen durchgeführt werden. In einer bevorzugten Ausführungsform von Verfahren (b) wird die Ausgangsverbindung der Formel (I) mit dem Verdün nungsmittel und dem Katalysator vermischt und dann so lange Wasserstoff zudosiert, bis kein Verbrauch von Wasserstoff mehr festzustellen ist. Nach dem Ende der Hydrierung filtriert man das Reaktionsgemisch und erhält nach Einengen des Filtrats das Rohprodukt als Rückstand, welches auf übliche Weise, beispielweise durch Säulenchromatographie, gereinigt werden kann.

Die beim erfindungsgemäßen Verfahren (c) zur Herstellung von Verbindungen der Formel (I) als Ausgangsstoffe zu verwendenden Verbindungen sind - mit der Maßgabe, daß jeweils $R^6$ für Amino steht - durch die Formel (I) allgemein definiert.

In Formel (I) haben dann $R^1$, $R^2$, $R^3$, $R^4$ $R^5$ und Az vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$, $R^5$ und Az angegeben wurden.

Die Ausgangsstoffe der Formel (I) für Verfahren (c) sind erfindungsgemäße, neue Verbindungen; sie können nach dem erfindungsgemäßen Verfahren (b) hergestellt werden.

Verfahren (c) wird unter Einsatz eines Hydrogenhalogenids bzw. von Schwefelsäure durchgeführt. Als Beispiele für die Hydrogenhalogenide seien Hydrogenfluorid, Hydrogenchlorid, Hydrogenbromid und Hydrogeniodid genannt.

Verfahren (c) wird gegebenenfalls unter Verwendung eines organischen Lösungsmittels durchgeführt. Geeignete organische Lösungsmittel sind beispielsweise Ether, wie Glycoldimethylether und Diglycoldimethylether, sowie Tetrahydrofuran und Dioxan, Ketone, wie Aceton und Methylethylketon, sowie Amide, wie Dimethylformamid.

Verfahren (c) wird unter Einsatz von Kupfer(I)-halogeniden bzw. von Kupfer(I)-cyanid durchgeführt. Als Beispiele für die Kupfer(I)-halogenide seien Kupfer(I)-chlorid, Kupfer(I)-bromid und Kupfer(I)-iodid genannt.

Die Reaktionstemperaturen können bei dem erfindungsgemäßen Verfahren (c) in einem größeren Bereich variiert werden. Im allgemeinen arbeitet man bei Temperaturen zwischen -20 °C und +80 °C, vorzugsweise bei Temperaturen zwischen 0 °C und 60 °C.

Das erfindungsgemäße Verfahren (c) wird im allgemeinen unter Normaldruck durchgeführt. Es ist jedoch auch möglich, unter erhöhtem oder vermindertem Druck zu abreiten.

Zur Durchführung des erfindungsgemäßen Verfahrens (c) setzt man je Mol Ausgangsverbindung der Formel (I) im allgemeinen zwischen 0,8 und 2,5 Mol, vorzugsweise zwischen 1,1 und 1,5 Mol, Natriumnitrit oder Kaliumnitrit und zwischen 2 und 20 Mol, vorzugsweise zwischen 3 und 10 Mol, Hydrogenhalogenid bzw. Schwefelsäure, sowie zwischen 1 und 3 Mol, vorzugsweise zwischen 1,1 und 1,5 Mol, Kupfer(I)-halogenid bzw. Kupfer(I)-cyanid ein.

Zunächst wird auf übliche Weise eine Diazotierung durchgeführt: Man legt hierzu im allgemeinen die Ausgangsverbindung der Formel (I) in einer wäßrigen Lösung eines Hydrogenhalogenids bzw. von Schwefelsäure vor und gibt dazu unter Kühlen langsam eine wäßrige Lösung von Natriumnitrit oder Kaliumnitrit. Überschüssige salpetrige Säure wird gegebenenfalls nach der Diazotierung mit Harnstoff beseitigt und die Diazoniumsalzlösung wird unter Kühlen zu einer wäßrigen Lösung eines Kupfer(I)-halogenids bzw. von Kupfer(I)-cyanid gegeben. Dann wird, gegebenenfalls unter Erwärmen, bis zum Ende der Gasentwicklung gerührt und anschließend nach üblichen Methoden aufgearbeitet.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoff ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen un Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao, Beerenfrucht- und Hopfenanlagen und zur selektiven Ünkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen, vor allem im Nachauflauf-Verfahren.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen

wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten und chlo rierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als fest Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau un organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4-(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Di chlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); N-Phosphonomethyl-glycin (GLYPHOSATE); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)acetanilid (MEFENACET); 2-{[[((4-Methoxy-6-methyl-1,3,5-triazin-2-yl)-amino)-carbonyl]-amino]-sulfonyl}-benzoesäure

oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

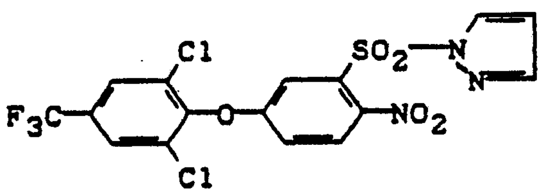

(Verfahren (a))

Eine Lösung von 6,0 g (0,013 Mol) 2-Nitro-5-(2,6-dichlor-4-trifluormethyl-phenoxy)-benzolsulfonsäurechlorid in 30 ml Methylenchlorid wird unter Rühren zu einer Mischung aus 0,9 g (0,013 Mol) Pyrazol, 1,3 g (0,016 Mol) Pyridin und 30 ml Methylenchlorid gegeben und das Reaktionsgemisch wid 15 Stunden bei 20 °C gerührt. Dann wird mit 2N-Salzsäure angesäuert, die organische Phase abgetrennt, mit Natriumsulfat getrocknet und filtriert. Vom Filtrat wird das Lösungsmittel im Wasserstrahlvakuum sorgfältig abdestilliert.

Man erhält 3,8 g (61 % der Theorie) (2-Nitro-5-(2,6-dichlor-4-trifluormethyl-phenoxy)-phenyl)-sulfonylpyrazol als kristallinen Rückstand vom Schmelzpunkt 159 °C.

### Beispiel 2

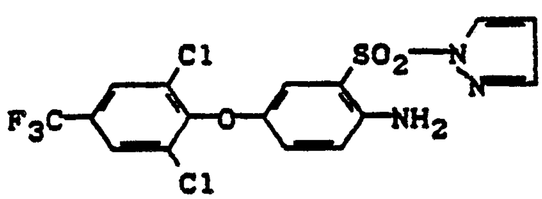

(Verfahren (b))

2,4 g (5 mMol) (2-Nitro-5-(2,6-dichlor-4-trifluormethyl-phenoxy)-phenyl)-sulfonylpyrazol werden in 100 ml Methanol gelöst und nach Zugabe von 2 g Raney-Nickel bei 30 °C unter 20 bis 30 bar Wasserstoffdruck hydriert. Dann wird filtriert, das Filtrat eingeengt und der Rückstand mit Isopropanol verrührt. Das hierbei kristallin angefallene Produkt wird durch Absaugen isoliert.

Man erhält 0,5 g (22 % der Theorie) (2-Amino-5-(2,6-dichlor-4-trifluormethyl-phenoxy)-phenyl)-sulfonylpyrazol vom Schmelzpunkt 232 °C.

Analog zu den Beispielen 1 und 2 sowie nach der allgemeinen Beschreibung der erfindungsgemäßen Herstellungsverfahren können die in der nachstehenden Tabelle 2 aufgeführten Verbindungen der Formel (I) erhalten werden.

Tabelle 2: Herstellungsbeispiele für die Verbindungen der Formel (I)

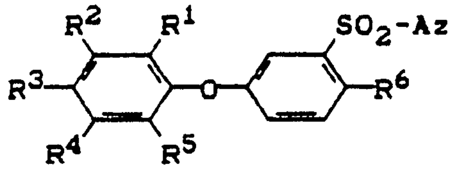

(I)

| Bsp.-Nr. | $R^1$ | $R^2$ | $R^3$ | $R^4$ | $R^5$ | $R^6$ | Az | Physikal. Daten |
|---|---|---|---|---|---|---|---|---|
| 3 | Cl | H | $CF_3$ | H | Cl | $NO_2$ | 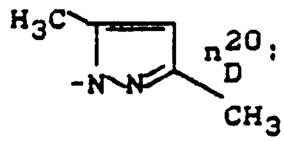 | $n_D^{20}$: 1,5611 |
| 4 | Cl | H | $CF_3$ | H | Cl | H | 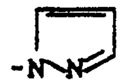 | Fp. 141 °C |
| 5 | Cl | H | $CF_3$ | H | Cl | H | 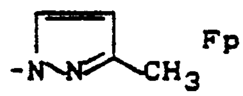 | Fp. 118 °C |
| 6 | Cl | H | $CF_3$ | H | H | H | 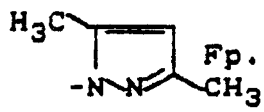 | Fp. 103 °C |
| 7 | Cl | H | $CF_3$ | H | Cl | $NO_2$ | 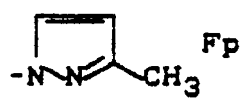 | Fp. 153 °C |
| 8 | Cl | H | $CF_3$ | H | Cl | $NO_2$ | 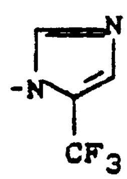 | Fp. 154 °C |
| 9 | Cl | H | $CF_3$ | H | F | $NO_2$ | 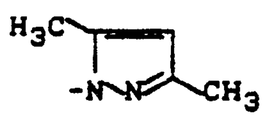 | |
| 10 | Cl | H | $CF_3$ | H | Cl | $NO_2$ | 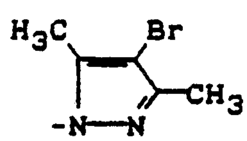 | |
| 11 | Cl | H | $CF_3$ | H | Cl | $NH_2$ | 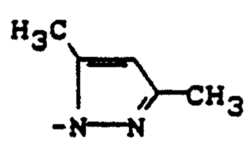 | Fp.163° C |

Ausgangsstoffe der Formel (II)

Beispiel (II-1)

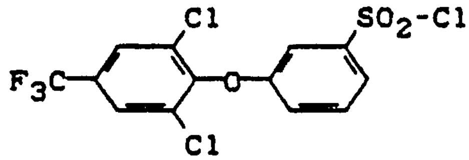

Eine Lösung von 34 g (0,49 Mol) Natriumnitrit in 50 ml Wasser wird unter Rühren und Kühlen auf 0 °C zu einer Mischung aus 145 g (0,45 Mol) 3-(2,6-Dichlor-4-trifluormethyl-phenoxy)-anilin, 120 ml Essigsäure und 160 ml konz. Salzsäure tropfenweise gegeben. Das Gemisch wird 90 Minuten bei 0 °C gerührt, nach Zugabe von 5 g Sulfamidsäure weitere 15 Minuten bei 0° C gerührt und dann zu 400 ml Essigsäure, welche man vorher mit Schwefeldioxid gesättigt hat, gegeben. Nach Zusatz von 9 g Kupfer(II)-chlorid - gelöst in 10 ml Wasser - wird das Gemisch 15 Stunden bei 10 °C bis 20 °C gerührt und dann auf Eis gegossen. Man extrahiert mit Methylenchlorid, trocknet die organische Phase mit Natriumsulfat und filtriert. Vom Filtrat wird das Lösungsmittel im Wasserstrahlvakuum sorgfältig abdestilliert.

Man erhält 108 g (59 % der Theorie) 3-(2,6-Dichlor-4-trifluormethyl-phenoxy)-benzolsulfonsäurechlorid als braungelben öligen Rückstand.

Beispiel (II-2)

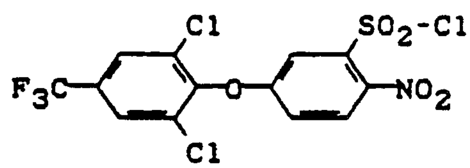

Eine Lösung von 3,0 g (0,05 Mol) Kaliumhydroxid in 7 ml Ethanol/2 ml Wasser wird tropfenweise unter Rühren zu einer Mischung aus 15,9 g (0,04 Mol) 1,2-Dinitro-4-(2,6-dichlor-4-(trifluormethyl-phenoxy)-benzol, 5,6 g (0,045 Mol) Benzylmercaptan und 140 ml Isopropanol gegeben und das Gemisch wird 3 Stunden unter Rückluß zum Sieden erhitzt. Das nach Erkalten kristallin angefallene Produkt wird durch Absaugen isoliert.

Man erhält 14,5 g (78,5 % der Theorie) 1-Nitro-2-benzylthio-4-(2,6-dichlor-4-trifluormethyl-phenoxy)-benzol vom Schmelzpunkt 207 °C.

Die gesamte Menge des so erhaltenen Produktes (14,5 g, 0,03 Mol) wird in 120 ml Essigsäure/20ml Wasser aufgenommen und bei 12 °C wird Chlor bis zur Sättigung eingeleitet. Man läßt das Reaktionsgemisch 15 Stunden bei 20 °C stehen, verdünnt dann mit Wasser, extrahiert mit Toluol, trocknet die organische Phase mit Natriumsulfat und filtriert. Vom Filtrat wird das Lösungsmittel im Wasserstrahlvakum sorgfältig abdestilliert.

Man erhält 12,5 g (93 % der Theorie) 2-Nitro-5-(2,6-dichlor-4-trifluormethyl-phenoxy)-benzolsulfonsäurechlorid als braungelben öligen Rückstand.

Anwendungsbeispiele

In den folgenden Anwendungsbeispielen wird die nachstehend aufgeführt Verbindung als Vergleichssubstanz herangezogen:

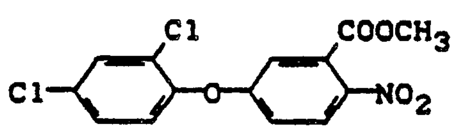

(A)

3-(2,4-Dichlor-phenoxy)-6-nitrobenzoesäure-methylester (bekannt aus US-PS 3 652 645 und US-PS 3 776 715).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

Eine deutliche Überlegenheit in der Wirksamkeit gegenüber der Vergleichssubstanz (A) zeigen in deisem Test z.B. die Verbindungen gemäß den Herstellungsbeispielen: (1), (3) und (7).

Beispiel B

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulagator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

In diesem Test zeigen beispielsweise die Wirkstoffe gemäß den Herstellungsbeispielen (1) und (3) ein sehr starkes Austrocknen der Blätter und sehr starken Blattfall.

## Ansprüche

1. Substituierte Phenoxyphenylsulfonylazole der Formel (I),

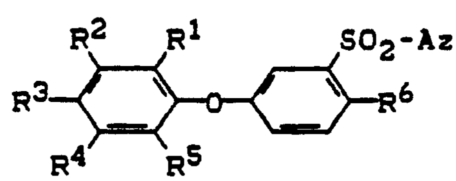

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,

$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls substituiertes Azolyl steht.

2. Substituierte Phenoxyphenylsulfonylazole der Formel (I) gemäß Anspruch 1, in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen-$C_1$-$C_4$-alkyl, Halogen-$C_1$-$C_4$-alkoxy und Halogen-$C_1$-$C_4$-alkylthio substituiertes Pyrazolyl, Imidazolyl oder Triazolyl steht.

3. Substituierte Phenoxyphenylsulfonylazole der Formel (I) gemäß Anspruch 1, in welcher
$R^1$ für Wasserstoff, Fluor, Chlor, Brom, Cyano oder Trifluormethyl steht,
$R^2$ für Wasserstoff, Fluor, Chlor oder Brom steht,
$R^3$ für Fluor, Chlor, Brom, Cyano, Trifluormethyl, Trifluormethoxy oder Trifluormethylsulfonyl steht,
$R^4$ für Wasserstoff, Fluor, Chlor oder Brom steht,
$R^5$ für Wasserstoff, Fluor, Chlor oder Brom steht,
$R^6$ für Wasserstoff, Fluor, Chlor, Brom, Iod, Cyano, Nitro oder Amino steht und
Az für gegebenenfalls einfach bis dreifach, gleich oder verschieden durch Halogen, Cyano, Nitro, Amino, $C_1$-$C_4$-Alkyl, $C_1$-$C_4$-Alkoxy, $C_1$-$C_4$-Alkylthio, Halogen $C_1$-$C_4$-alkyl, Halogen-$C_1$-$C_4$-alkoxy und Halogen-$C_1$-$C_4$-alkylthio substituiertes Pyrazolyl, Imidazolyl oder Triazolyl steht.

4. Verfahren zur Herstellung von substituierten Phenoxyphenylsulfonylazolen der Formel (I),

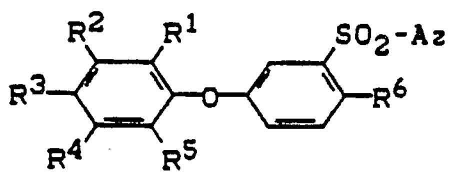

(I)

in welcher
$R^1$ für Wasserstoff, Halogen, Cyano oder Trifluormethyl steht,
$R^2$ für Wasserstoff oder Halogen steht,
$R^3$ für Halogen, Cyano, Trifluormethyl, Trifluormethoxy oder Trifluormethylsulfonyl steht,
$R^4$ für Wasserstoff oder Halogen steht,
$R^5$ für Wasserstoff oder Halogen steht,
$R^6$ für Wasserstoff, Halogen, Cyano, Nitro oder Amino steht und
Az für gegebenenfalls substituiertes Azolyl steht,
dadurch gekennzeichnet, daß man

(a) für den Fall, daß in Formel (I) $R^6$ für Wasserstoff oder Nitro steht und $R^1$, $R^2$, $R^3$, $R^4$, $R^5$ und Az die oben angegebenen Bedeutungen haben,
substituierte Phenoxybenzolsulfonsäurechloride der allgemeinen Formel (II)

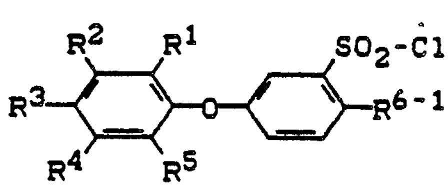

(II)

in welcher
$R^{6-1}$ für Wasserstoff oder Nitro steht und
$R^1$, $R^2$, $R^3$, $R^4$ und $R^5$ die oben angegebenen Bedeutungen haben,
mit Azolen der allgemeinen Formel (III)
H - Az (III)

in welcher
Az die oben angegebene Bedeutung hat,
oder mit Alkalimetallsalzen von Azolen der Formel (III)
gegebenenfalls in Gegenwart eines Säureakzeptors und gegebenenfalls in Gegenwart eines Verdünnungsmittels umsetzt, oder daß man

(b) für den Fall, daß in Formel (I) $R^6$ für Amino steht und $R^1$, $R^2$, $R^3$, $R^4$, $R^5$ und Az die oben angegebenen Bedeutungen haben,
Verbindungen der allgemeinen Formel (I), in welcher $R^6$ für Nitro steht und $R^1$, $R^2$, $R^3$, $R^4$, $R^5$ und Az die oben angegebenen Bedeutungen haben,
mit Wasserstoff in Gegenwart eines Hydrierungskatalysators und in Gegenwart eines Verdünnungsmittels umsetzt, oder daß man

(c) für den Fall, daß in Formel (I) $R^6$ für Halogen oder Cyano steht und $R^1$, $R^2$, $R^3$, $R^4$, $R^5$ und Az die oben angegebenen Bedeutungen haben,
Verbindungen der allgemeinen Formel (I), in welcher $R^6$ für Amino steht und $R^1$, $R^2$, $R^3$, $R^4$, $R^5$ und Az die oben angegebenen Bedeutungen haben,
mit Natriumnitrit oder Kaliumnitrit und mit einem Hydrogenhalogenid (für $R^6$: Halogen) bzw. mit Schwefelsäure (für $R^6$: Cyano) in Gegenwart von Wasser und gegebenenfalls in Gegenwart eines organischen Lösungsmittels umsetzt und die hierbei erhaltenen Diazoniumsalzlösungen mit in Wasser gelösten Kupfer-(I)-halogeniden bzw. mit Kupfer(I)-cyanid umsetzt.

5. Herbizide und pflanzenwuchsregulierende Mittel, gekennzeichnet durch einen Gehalt an mindestens einem substituierten Phenoxyphenylsulfonylazol der Formel (I) gemäß den Ansprüchen 1 bis 4.

6. Verfahren zur Bekämpfung von Unkräutern und zur Regulierung des Pflanzenwachstums, dadurch gekennzeichnet, daß man substituierte Phenoxyphenylsulfonylazole der Formel (I) gemäß den Ansprüchen 1 bis 4 auf die Unkräuter bzw. Pflanzen und/oder ihren Lebensraum einwirken läßt.

7. Verwendung von substituierten Phenoxyphenylsulfonylazolen der Formel (I) gemäß den Ansprüchen 1 bis 4 zur Bekämpfung von Unkräutern und/oder als Pflanzenwuchsregulatoren.

8. Verfahren zur Herstellung von herbiziden und/oder pflanzenwuchsregulierenden Miteln, dadurch gekennzeichnet, daß man substituierte Phenoxyphenylsulfonylazole der Formel (I) gemäß den Ansprüchen 1 bis 4 mit Streckmitteln und/oder oberflächenaktiven Substanzen vermischt.

Europäisches Patentamt

# EUROPÄISCHER RECHERCHENBERICHT

Nummer der Anmeldung

EP 89 11 7180

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 238 824 (BAYER AG)<br>--- | | C 07 D 233/56<br>A 01 N 43/56<br>C 07 D 231/16<br>A 01 N 43/50<br>A 01 N 43/653<br>C 07 D 249/08 |
| A | EP-A-0 237 912 (NIHON TOKUSHU NOYAKU SEIZO K.K.)<br>--- | | |
| A | EP-A-0 173 918 (NIHON TOKUSHU NOYAKU SEIZO K.K.)<br>--- | | |
| A | EP-A-0 044 394 (BASF AG)<br>--- | | |
| D,A | US-A-3 776 715 (R.J. THEISSEN)<br>--- | | |
| D,A | US-A-3 652 645 (R.J. THEISSEN)<br>----- | | |

RECHERCHIERTE SACHGEBIETE (Int. Cl.5)

C 07 D 233/00
A 01 N 43/00
C 07 D 231/00
C 07 D 249/00

Der vorliegende Recherchenbericht wurde für alle Patentansprüche erstellt

| Recherchenort | Abschlußdatum der Recherche | Prüfer |
|---|---|---|
| DEN HAAG | 12-12-1989 | DE BUYSER I.A.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument